# FASCICULE DE BREVET EUROPEEN

(11) **EP 0 677 288 B1**
(45) Date de publication et mention de la délivrance du brevet: **28.01.1998**
(21) Numéro de dépôt: 95400443.8
(22) Date de dépôt: 01.03.1995
(51) Int. Cl.: A61K 7/48

(54) **Composition cosmétique ou pharmaceutique contenant un carbamate d'alkyle en tant qu'agent hydratant**
Kosmetische oder pharmazeutische Zuzammensetzung, die ein Alkylkarbamat als Hydratisierungsmittel enthält
Cosmetic or pharmaceutic composition comprising an alkyl-carbamate as moisturizing agent

(30) Priorité: 13.04.1994 FR 9404377
(43) Date de publication de la demande: 18.10.1995
(73) Titulaire: L'OREAL, 75008 Paris (FR)
(72) Inventeur: Philippe, Michel, F-91320 Wissous (FR); Semeria, Didier, F-77181 Courtry (FR); Bollens, Eric, F-94410 Saint Maurice (FR)
(74) Mandataire: Dodin, Catherine

(56) Documents cités:
- EP-A- 0 408 448
- EP-A- 0 420 722
- EP-A- 0 450 527
- EP-A- 0 577 506
- BE-A- 543 132
- FR-A- 2 379 283
- FR-A- 2 703 993
- US-A- 2 040 997
- US-A- 2 808 402

## Description

La présente invention concerne l'utilisation comme agent hydratant, dans et pour la préparation d'une composition cosmétique ou pharmaceutique, d'un N-(hydroxyalkyl)carbamate d'alkyle.

On connait, selon la demande EP-A-577 506, l'utilisation de carbamates à chaîne alkyle linéaire comme agent épaississant dans des solutions d'agents tensioactifs.

D'autres dérivés carbamates sont également connus de l'état de la technique comme agents hydratants. Par exemple, la demande EP-A-420 722 décrit des composés lipidiques dérivés de sphingosines; des dérivés d'uréthanes comprenant au moins un groupement acide carboxylique ont aussi été décrits dans la demande EP-A-408 448; des esters d'acides carbamiques sont également mentionnés dans la demande FR-A-2379283.

La Demanderesee a découvert de manière surprenante une nouvelle famille de composés dérivés de carbamates présentant des propriétés d'hydratation de la peau. Ces composés répondent à la formule générale (I) suivante: 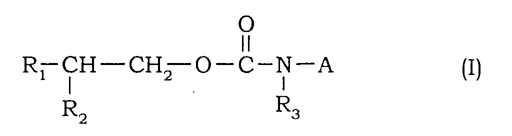 dans laquelle:
- R₁ représente un radical alkyle ou alkylène ayant 4-18 atomes de carbone,
- R₂ représente un radical alkyle ou alkylène ayant 2-16 atomes de carbone,
- R₃ représente un atome d'hydrogène ou un radical alkyle ayant 1-6 atomes de carbone, et
- A représente un groupement hydrophile non ionique.

Ces composés peuvent être obtenus par réaction, dans un solvant, d'un aminoalcool de formule R₃-NH-A avec un composé de formule (II) :

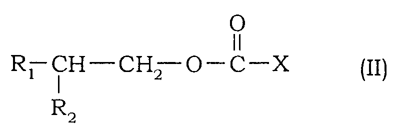

R₁, R₂, R₃, A ayant les même significations que celles données ci-dessus et X représentant un atome d'halogène, notamment un atome de chlore, ou un radical dérivé d'un azole, notamment un radical provenant d'un imidazole tel que celui de formule (III) :

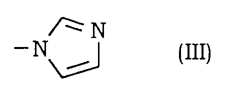

Comme solvant, on peut utiliser le dichlorométhane, le dichloro-1,2-éthane, le trichloro-1,1,1-éthane, le chloroforme, l'acétonitrile, le toluène, le dioxane, le tétrahydrofuranne, le diméthoxy-1,2-éthane, le cyclohexane, l'eau ou un mélange de ces solvants.
La réaction est effectuée à une température comprise entre -5°C et 50°C, de préférence inférieure à 10°C.
La réaction peut être effectuée en présence d'une base. Celle-ci peut être choisie parmi les hydroxydes de métaux alcalins ou alcalino-terreux, l'hydrogénocarbonate de sodium, les alcoolates de métaux alcalins, les hydrures alcalins, les amines tertiaires telles que la pyridine ou la triéthylamine.

Parmi les composés correspondant à la formule générale (I), on peut notamment citer :
- la N-2-éthylhexyloxycarbonyl-N-méthyl-D-glucamine,
- la N-2-butyloctyloxycarbonyl-N-méthyl-D-glucamine,
- la N-2-décyltétradécyloxycarbonyl-D-glucamine,
- la N-2-hexyldécyloxycarbonyl-D-glucamine,
- le 3-N-(2-décyltétradécyloxycarbonyl)-amino-1,2-propanediol,
- le 2-N-(2-décyltétradécyloxycarbonyl)-amino-2-hydroxyméthyl-1,3-propanediol, et
- le 2-N-(2-hexyldécyloxycarbonyl)-amino-1-éthanol.

On a constaté que, de manière surprenante, les composés de formule (I) présentent des propriétés cosmétiques intéressantes, en particulier des propriétés d'hydratation de la peau.
On a en effet constaté qu'il est possible, en appliquant ces produits sur la peau, d'en diminuer la perte en eau et/ou d'augmenter la fixation d'eau dans le stratum corneum. Ces composés peuvent donc être utilisés comme agents hydratants, notamment de la peau, chez l'humain. Ils permettent de conserver ou de restaurer la souplesse de la peau, son élasticité, sa résistance aux mouvements du corps et sa fonction de barrière à l'entrée de substances toxiques.

La présente invention a donc pour objet l'utilisation d'un composé de formule (I) en tant qu'agent hydratant dans une et pour la préparation d'une composition, notamment cosmétique ou pharmaceutique.

Dans le composé de formule (I) selon l'invention, le radical R1 peut représenter un radical alkyle ayant de 4 à 14 atomes de carbone, R2 peut représenter un radical alkyle ayant de 2 à 12 atomes de carbone et R3 peut représenter un atome d'hydrogène ou un radical méthyle.
De préférence, A représente un radical - (CH₂)ₙ-(CHOH)ₘ-Z, dans lequel n représente un nombre entier égal à 0 ou 1, m représente un nombre entier compris entre 0 et 5 et Z est un radical alkyle monohydroxylé ou polyhydroxylé ayant de 1 à 4 atomes de carbone.

En particulier, Z est choisi dans le groupe comprenant les radicaux suivants :

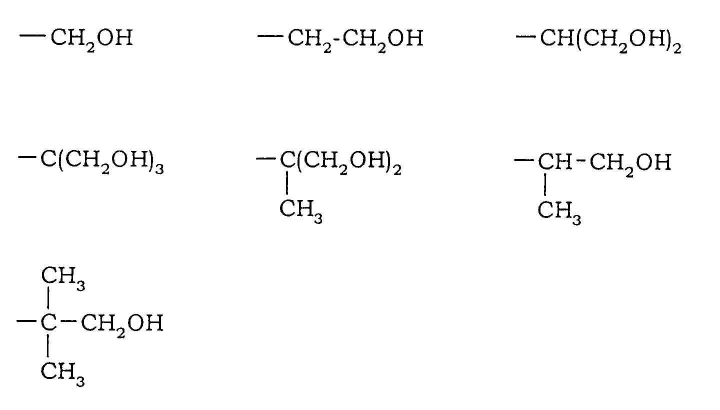

La composition cosmétique selon l'invention comprend généralement, en plus de l'agent hydratant selon l'invention, un véhicule cosmétiquement acceptable tel que l'eau; les solvants organiques compatibles avec une application cutanée tels que l'acétone, l'isopropanol, I'éthanol; les triglycérides d'acides gras à 6-24 atomes de carbone, les éthers de glycol, les esters de polyalkylèneglycols et les silicones volatiles.
La composition cosmétique selon l'invention comprend de préférence 0,001 à 15% en poids de composé de formule (I) par rapport au poids total de la composition.
La composition selon l'invention peut également contenir des additifs cosmétiques conventionnels tels que des corps gras, notamment les huiles naturelles ou synthétiques; des agents épaississants ou gélifiants tels que la cellulose ou ses dérivés, les polymères acryliques, les alginates, les gommes, les polyéthylèneglycols, les bentonites et les montmorillonites; des agents humectants tels que la glycérine et la triacétine; des agents antioxydants; des conservateurs.

La composition selon l'invention peut se présenter sous forme de lotion aqueuse, de gel, d'émulsion, de crème ou de mousse à appliquer sur la peau. Elle peut également se présenter sous forme de dispersion vésiculaire de lipides ioniques ou non ioniques, lesdites vésicules pouvant alors servir en tant qu'agent d'encapsulation pour des ingrédients actifs lipophiles ou hydrophiles tels que l'acide rétinoïque ou des agents filtres UV.
Ladite composition peut être utilisée en tant que produit de soin et/ou produit de maquillage, en particulier en tant que crème de soin ou solaire, fond de teint et/ou fard à joues ou à paupières.

L'invention est illustré plus en détail dans les exemples suivants dans lesquels les pourcentages sont donnés en poids sauf indication contraire.

Les exemples 1 à 7 décrivent un mode de préparation de sept composés particuliers selon l'invention et les exemples 8 à 12 illustrent les propriétés hydratantes des composés selon l'invention et des formulations de compositions cosmétiques les contenant.
Auparavant, sont décrites les deux méthodes employées pour évaluer l'action des composés en tant qu'agents hydratants de la peau.

### A) Mesure de la perte insensible en eau (PIE)

Cette mesure s'effectue à l'aide d'un évaporimètre (Servomed) qui détermine quantitativement l'évaporation d'eau, c'est-à-dire un transport d'eau par diffusion, à partir d'un échantillon de stratum corneum obturant une capsule cylindrique contenant de l'eau, le tout étant placé dans une chambre à température et humidité relatives contrôlées.
Des capteurs permettent de mesurer la pression partielle de vapeur d'eau en deux points situés à des distances différentes de l'échantillon.
On détermine ainsi le gradient de pression partielle de vapeur d'eau entre les deux points, et donc le taux d'évaporation conformément à la loi de Fick.

### B) Mesure de la conductivité de la peau

Cette mesure permet de mettre en évidence la variation du taux d'hydratation de la peau.
Elle est effectuée à l'aide d'un appareil comportant une électrode centralesous forme de tige entourée par une électrode cylindrique.
L'appareil est appliqué sur la peau et l'on applique un courant alternatif de haute fréquence.
On constate que plus l'hydratation de la peau est importante, plus la quantité de courant consommé est élevée.
On met ainsi en évidence l'augmentation de la conductivité de la peau et donc l'augmentation de son taux d'hydratation.

### Exemple 1 : Préparation de la N-2-éthylhexyloxycarbonyl-N-méthyl-D-glucamine

Dans un réacteur, on dissout 117 g (0,6 mole) de N-méthyl-D-glucamine dans un mélange de 800 ml d'eau et 400 ml de tétrahydrofurane, puis on disperse 201,6 g (2,4 moles) d'hydrogénocarbonate de sodium. En maintenant la température du mélange réactionnel à 5°C, on ajoute goutte-à-goutte 115,6 g (0,6 mole) de chloroformiate de 2-éthylhexyle puis on laisse réagir 3 heures sous agitation à 5°C et une nuit au repos à la température ambiante. Le mélange réactionnel est alors filtré et concentré; le résidu pâteux obtenu est dissout dans 2 litres d'acétone puis filtré après refroidissement à 5°C. Le produit cristallisé recueilli est séché. On obtient 105 g (rendement 50 %) de N-2-éthylhexyloxycarbonyl-N-méthyl-D-glucamine, dont le point de fusion est de 74°C.

### Exemple 2 : Préparation de la N-2-butyloctyloxycarbonyl-N-méthyl-D-glucamine

Le composé est préparé selon le même mode opératoire que l'exemple 1, en utilisant :
- 78 g (0,4 mole) de N-méthyl-D-glucamine
- 134,4 g (1,6 mole) de bicarbonate de soude
- 99,4 g (0,4 mole) de chloroformiate de 2-butyloctyle.

On obtient 62 g de N-2-butyloctyloxycarbonyl-N-méthyl-D-glucamine sous forme d'une poudre blanche dont le point de fusion est 77°C.

### Exemple 3 : Préparation de la N-2-décyltétradécyloxycarbonyl-D-glucamine

Dans un réacteur de 1000 ml, on solubilise 36,2 g de D-glucamine dans 100 ml d'eau. On ajoute 135 ml de tétrahydrofurane puis 67,2 g de bicarbonate de soude. Sous agitation, on porte le mélange à -2°C puis on additionne goutte à goutte 83,3 g de chloroformiate de 2-décyltétradécyle. A la fin de l'addition, on laisse le mélange deux heures à 0°C puis on le laisse revenir progressivement en deux heures également à 25°C. On élimine sur fritté l'insoluble puis on concentre à sec à l'évaporateur rotatif. Le résidu est recristallisé dans un litre d'acétone, cette opération est renouvelée une fois dans un litre d'acétone puis une fois dans un litre de mélange acétate d'éthyle/acétone.
On obtient 61 g de N-2-décyltétradécyloxycarbonyl-D-glucamine sous forme d'une poudre blanche dont le point de fusion est de 82°C.

### Exemple 4 : Préparation de la N-2-hexyldécyloxycarbonyl-D-glucamine

Le composé est préparé selon le même mode opératoire que l'exemple 3, en utilisant :
- 46,8 g (0,24 mole) de D-glucamine,
- 80,6 g (0,96 mole) de bicarbonate de soude,
- 73,08 g (0,24 mole) de chloroformiate de 2-hexyldécyle.

On obtient 70 g de N-2-hexyldécyloxycarbonyl-D-glucamine sous forme d'une poudre blanche dont le point de fusion est de 81°C.

### Exemple 5 : Préparation du 3-N-(2-décyltétradécyloxycarbonyl)-amino-1,2-propanediol

Le composé est préparé selon le mode opératoire de l'exemple 3, en utilisant:
- 18,2 g (0,2 mole) de 3-amino-1,2-propanediol,
- 67,2 g (0,8 mole) de bicarbonate de soude,
- 83,3 g (0,2 mole) de chloroformiate de 2-décyltétradécyle.

On obtient 95 g d'une huile ambrée qui est purifiée sur silice à l'aide de mélanges dichlorométhane/méthanol (de 10/0 à 9/1 vol/vol).
On obtient 80 g d'une cire blanche dont la pureté est contrôlée par chromatographie sur couche mince (éluant dichlorométhane/méthanol : 95/5 vol/vol Rf = 0,34)

### Exemple 6 : Préparation du 2-N-(2-décyltétradécyloxycarbonyl)-amino-2-hydroxyméthyl-1,3-propanediol

Le composé est préparé selon le mode opératoire de l'exemple 3, en utilisant :
- 24,2 g (0,2 mole) de tris(hydroxyméthyl)aminométhane,
- 67,2 g (0,8 mole) de bicarbonate de sodium,
- 83,3 g (0,2 mole) de chloroformiate de 2-décyltétradécyle.

On obtient, après réaction et purification sur silice (éluant heptane/acétate d'éthyle : 7/3 puis 6/4 vol/vol), 40 g d'une cire blanche dont le point de fusion est de 51°C et dont la pureté est vérifiée par chromatographie sur couche mince (dichlorométhane/méthanol : 9/1 vol/vol ; Rf = 0,45).

### Exemple 7 : Préparation du 2-N-(2-hexyldécyloxycarbonyl)-amino-1-éthanol

On solubilise 105,3 g de carbonyldiimidazole (0,65 mole) dans 1200 ml de dichlorométhane. On ajoute ensuite goutte à goutte en 1 heure à 20°C, 133,38 g (0,55 mole) de 2-hexyldécanol. On laisse agiter 4 heures à 20°C. Le mélange réactionnel est versé dans une ampoule à décanter et lavé 4 fois avec 200 ml d'eau. La phase organique est séchée sur sulfate de sodium puis concentrée à sec.
On obtient 173 g de 2-hexyldécyloxycarbonyl imidazole sous la forme d'une huile jaune.
On solubilise 12,2 g (0,2 mole) d'aminoéthanol dans 400 ml de tétrahydrofurane anhydre. On ajoute à 20°C une solution de 73,9 g (0,22 mole) de 2-hexyldécyloxycarbonyl imidazole dans 300 ml de tétrahydrofurane en 90 minutes.
On poursuit l'agitation pendant 24 heures à 20°C puis on évapore à sec. On obtient 88 g d'une huile qui est solubilisée dans 500 ml d'acétate d'éthyle puis lavée trois fois avec 100 ml d'eau. Les phases organiques sont regroupées, séchées sur sulfate de sodium puis évaporées à sec.
On obtient 71 g d'une huile ambrée qui est purifiée sur silice (éluant heptane/ acétate d'éthyle 9/1 puis 8/2 vol/vol) pour conduire à 35 g d'une huile incolore dont la pureté est contrôlée par chromatographie sur couche mince (éluant acétate d'éthyle/heptane 5/5 vol/vol ; Rf = 0,55).

### Exemple 8

Les composés des exemples 2, 3, 4, 6 et 7 sont préparés à raison de 3% dans l'isopropanol, puis appliqués sur des morceaux de stratum corneum délipidé.
On mesure, 20 heures après l'application, la perte insensible en eau (PIE) pour chaque composé.
On obtient les résultats suivants :

| Composé | 2 | 3 | 4 | 6 | 7 |
|---|---|---|---|---|---|
| P.I.E. | - 6 +/- 1 | - 26 +/- 7 | - 25 +/- 2 | - 29 +/- 3 | - 8 +/- 2 |

On constate donc que l'application des composés selon l'invention permet de réduire de manière significative l'évaporation de l'eau contenue dans le stratum corneum.

### Exemple 9

Les composés des exemples 1, 3, 4, 5 et 6 sont préparés à raison de 3% dans l'isopropanol, puis appliqués sur des morceaux de stratum corneum délipidé.
On mesure, 20 heures après l'application, la conductivité du stratum corneum pour chaque composé.
On obtient les résultats suivants :

| Composé | 1 | 3 | 4 | 5 | 6 |
|---|---|---|---|---|---|
| Conductivité | 37 +/- 15 | 60 +/-25 | 41 +/-7 | 43 +/- 6 | 65 +/-8 |

On constate que la conductivité de la peau est augmentée lorsqu'elle est traitée avec les composés selon l'invention. Ces composés permettent donc bien d'en augmenter le taux d'hydratation.

### Exemple 10 - Exemple comparatif

On effectue une mesure de conductivité sur deux morceaux de stratum corneum non délipidé, sur lesquels on a appliqué le composé à 3% dans l'isopropanol et qu'on a laissé reposer pendant 20 heures.
Cette mesure est effectuée pour
a) le 2-N-(2-décyltétradécyloxycarbonyl)-amino-2-hydroxyméthyl-1,3-propanediol (composé de l'exemple 6), et
b) le 2-N-(2-décyltétradécanoyl)-amino-2-hydroxyméthyl-1,3-propanediol, connu pour être un agent hydratant.

On obtient les résultats suivants :

| | Conductivité |
|---|---|
| Composé a) selon l'invention | 23 +/- 4 |
| Composé b) comparatif | non significatif |

Le composé selon l'invention permet donc d'augmenter le taux d'hydratation de la peau de manière plus significative que le composé de l'état de la technique, pourtant connu pour être un agent hydratant.

### Exemple 11

On prépare une crème de jour pour le visage ayant la composition suivante:

| | |
|---|---|
| . alcool cétylique | 2,5% |
| . tristéarate de sorbitan | 0,9% |
| . stéarate de polyoxyde d'éthylène (polyoxyéthyléné 40 fois) | 2% |
| . stéarate de glycéryle | 3% |
| . myristate de myristyle | 2% |
| . polyisobutène hydrogéné | 2,5% |
| . palmitate d'octyle | 4% |
| . polydiméthylsiloxane (10 cm².s) | 5% |
| . huile de noyaux d'abricot | 5,7% |
| . N-2-décyltétradécyloxycarbonyl-D-glucamine | 0,5% |
| . conservateurs | 0,5% |
| . eau | qsp 100% |

On obtient une crème de jour se présentant sous forme d'une émulsion permettant de bien couvrir et protéger la peau, particulièrement adaptée pour les peaux normales et sèches.

### Exemple 12

On prépare une crème de nuit ayant la formulation suivante:

| | |
|---|---|
| . mélange de mono- et di-stéarate de glycéryle et de stéarate de polyoxyde d'éthylène | 2% |
| . 2-N-(2-décyltétradécyloxycarbonyl)-amino-2-hydroxyméthyl-1,3-propanediol | 3% |
| . huile de noyaux d'abricot | 17% |
| . cyclopentadiméthylsiloxane | 1,5% |
| . carbomer (vendu sous la marque CARBOPOL) | 0,75% |
| . conservateurs | 0,5% |
| . triéthanolamine | 0,75% |
| . eau | qsp 100% |

On obtient une crème de nuit, se présentant sous forme d'une émulsion épaisse, brillante et très douce à appliquer, qui se révèle nourrissante et hydratante pour la peau, particulièrement pour les peaux sèches.

## Revendications

1. Utilisation dans et pour la préparation d'une composition cosmétique ou pharmaceutique comprenant au moins un composé répondant à la formule générale (I) : 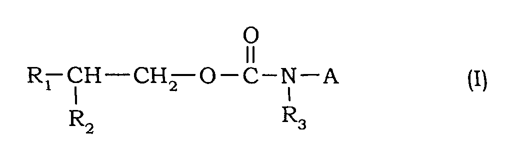 dans laquelle:
- R₁ représente un radical alkyle ou alkylène ayant de 4 à 18 atomes de carbone,
- R₂ représente un radical alkyle ou alkylène ayant de 2 à 16 atomes de carbone,
- R₃ représente un atome d'hydrogène ou un radical alkyle ayant de 1 à 6 atomes de carbone,
- A représente un radical -(CH₂)ₙ-(CHOH)ₘ-Z, dans lequel n représente un nombre entier égal à 0 ou 1, m représente un nombre entier compris entre 0 et 5 et Z est un radical alkyle monohydroxylé ou polyhydroxylé ayant de 1 à 4 atomes de carbone,
comme agent hydratant.

2. Utilisation selon la revendication 1, caractérisée par le fait que R1 représente un radical alkyle ayant de 4 à 14 atomes de carbone, R2 représente un radical alkyle ayant de 2 à 12 atomes de carbone et R3 représente un atome d'hydrogène ou un radical méthyle.

3. Utilisation selon la revendication 1 ou 2, caractérisée par le fait que Z représente un radical hydroxylé choisi dans le groupe comprenant les radicaux suivants :

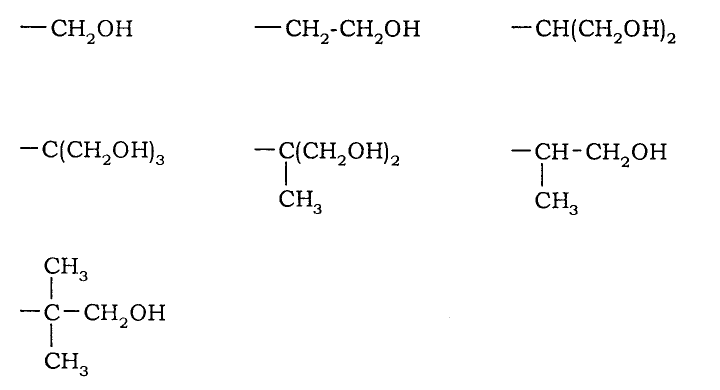

4. Utilisation selon l'une des revendications précédentes, caractérisée par le fait que le composé de formule (I) est choisi parmi :
- la N-2-éthylhexyloxycarbonyl-N-méthyl-D-glucamine,
- la N-2-butyloctyloxycarbonyl-N-méthyl-D-glucamine,
- la N-2-décyltétradécyloxycarbonyl-D-glucamine,
- la N-2-hexyldécyloxycarbonyl-D-glucamine,
- le 3-N-(2-décyltétradécyloxycarbonyl)-amino-1,2-propanediol,
- le 2-N-(2-décyltétradécyloxycarbonyl)-amino-2-hydroxyméthy-1,3-propanediol, et
- le 2-N-(2-hexyldécyloxycarbonyl)-amino-1-éthanol

5. Utilisation selon l'une des revendications précédentes, caractérisée par le fait que le composé de formule (I) est présent à une concentration comprise entre 0,001 et 15 % en poids par rapport au poids total de la composition.

6. Utilisation selon l'une des revendications précédentes, caractérisée par le fait que la composition se présente sous la forme d'un produit de soin tel qu'une crème de soin ou solaire ou d'un produit de maquillage tel qu'un fond de teint, un fard à joues ou un fard à paupières.

## Claims

1. Use in, and for the preparation of, a cosmetic or pharmaceutical composition comprising at least one compound corresponding to the general formula (I): 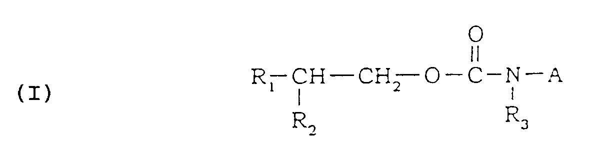 in which:
- R₁ represents an alkyl or alkenyl radical having from 4 to 18 carbon atoms,
- R₂ represents an alkyl or alkenyl radical having from 2 to 16 carbon atoms,
- R₃ represents a hydrogen atom or an alkyl radical having from 1 to 6 carbon atoms, and
- A represents a radical -(CH₂)ₙ-(CHOH)ₘ-Z, in which n represents an integer equal to 0 or 1, m represents an integer between 0 and 5 and Z is a monohydroxylated or polyhydroxylated alkyl radical having from 1 to 4 carbon atoms, as hydrating agent.

2. Use according to Claim 1, characterized in that R₁ represents an alkyl radical having from 4 to 14 carbon atoms, R₂ represents an alkyl radical having from 2 to 12 carbon atoms and R₃ represents a hydrogen atom or a methyl radical.

3. Use according to Claim 1 or 2, characterized in that Z represents a hydroxylated radical chosen from the group comprising the following radicals:

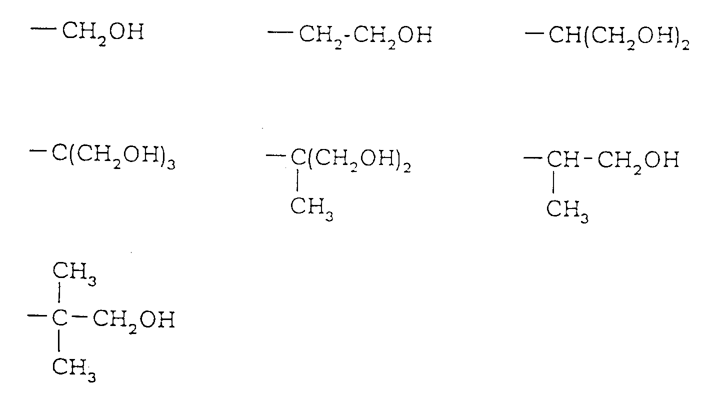

4. Use according to one of the preceding claims, characterized in that the compound of the formula (I) is chosen from:
- N-(2-ethylhexyloxycarbonyl)-N-methyl-D-glucamine,
- N-(2-butyloctyloxycarbonyl)-N-methyl-D-glucamine,
- N-(2-decyltetradecyloxycarbonyl)-D-glucamine,
- N-(2-hexyldecyloxycarbonyl)-D-glucamine,
- 3-[N-(2-decyltetradecyloxycarbonyl)amino]-1,2-propanediol,
- 2-[N-(2-decyltetradecyloxycarbonyl)amino]-2-hydroxymethyl-1,3-propanediol, and
- 2-[N-(2-hexyldecyloxycarbonyl)amino]-1-ethanol.

5. Use according to one of the preceding claims, characterized in that the compound of the formula (I) is present at a concentration of between 0.001 and 15 % by weight relative to the total weight of the composition.

6. Use according to one of the preceding claims, characterized in that the composition takes the form of a skin care product such as a skin care or sun cream, or the form of a make-up product such as a make-up foundation, a blusher or an eyeshadow.

## Patentansprüche

1. Verwendung von Verbindungen, die der allgemeinen Formel (I): 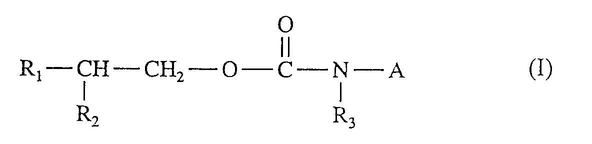 entsprechen, in der
- R₁ eine Alkyl- oder Alkenylgruppe mit 4 bis 18 Kohlenstoffatomen darstellt
- R₂ eine Alkyl- oder Alkenylgruppe mit 2 bis 16 Kohlenstoffatomen darstellt
- R₃ ein Wasserstoffatom oder eine Alkylgruppe mit 1 bis 6 Kohlenstoffatomen darstellt und
- A eine Gruppe -(CH₂)ₙ-(CHOH)ₘ-Z darstellt, in der n gleich 0 oder 1 ist, m eine ganze Zahl von 0 bis 5 darstellt und Z eine mono- oder polyhydroxylierte Alkylgruppe mit 1 bis 4 Kohlenstoffatomen darstellt
als Hydratisierangsmittel in kosmetischen oder pharmazeutischen Zusammensetzungen sowie für deren Herstellung, wobei diese Zusammensetzungen mindestens eine der Formel (I) entsprechende Verbindung enthalten.

2. Verwendung nach Anspruch 1, dadurch gekennzeichnet, daß R₁ eine Alkylgruppe mit 4 bis 14 Kohlenstoffatomen, R₂ eine Alkylgruppe mit 2 bis 12 Kohlenstoffatomen und R₃ ein Wasserstoffatom oder eine Methylgruppe darstellt.

3. Verwendung nach Anspruch 1 oder 2, dadurch gekennzeichnet, daß Z eine hydroxylierte Gruppe darstellt, die ausgewählt ist unter den folgenden Gruppen:

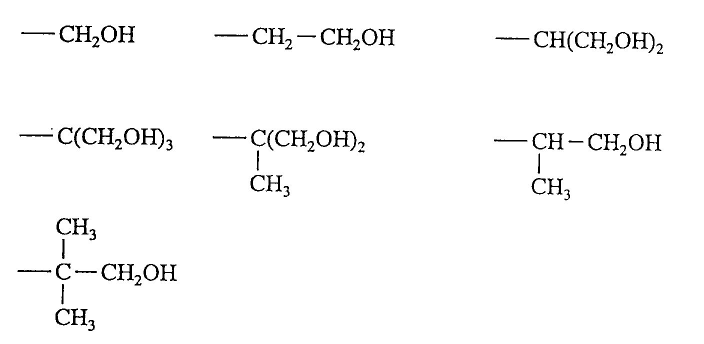

4. Verwendung nach einem der vorhergehenden Ansprüche, dadurch gekennzeichnet, daß die Verbindung der Formel (I) ausgewählt ist unter:
- N-(2-Ethylhexyloxycarbonyl)-N-methyl-D-glucamin,
- N-(2-Butyloctyloxycarbonyl)-N-methyl-D-glucamin, N-(2-Decyltetradecyloxycarbonyl)-D-glucamin,
- N-(2-Hexyldecyloxycarbonyl)-D-glucamin,
- 3-N-(2-Decyltetradecyloxycarbonyl)-amino-1,2-propandiol,
- 2-N-(2-Decyltetradecyloxycarbonyl)-amino-2-hydroxymethyl-1,3-propandiol,
und
- 2-N-(2-Hexyldecyloxycarbonyl)-amino-1-ethanol.

5. Verwendung nach einem der vorhergehenden Ansprüche, dadurch gekennzeichnet, daß die Verbindung der Formel (I) in einer Konzentration von 0,001 bis 15 Gew.-%, bezogen auf das Gesamtgewicht der Zusammensetzung, vorliegt.

6. Verwendung nach einem der vorhergehenden Ansprüche, dadurch gekennzeichnet, daß die Zusammensetzung in Form eines Pflegeprodukts wie einer Pflege- oder Sonnencreme oder eines Schminkprodukts wie eines Make-ups, eines Wangenrouges oder eines Lidschattens vorliegt.
